Europäisches Patentamt

European Patent Office

Office européen des brevets

(11) Publication number: **0 502 236 A1**

## EUROPEAN PATENT APPLICATION

(21) Application number: **91107278.3**

(22) Date of filing: **06.05.91**

(51) Int. Cl.⁵: **C12N 15/34**, A01N 63/00, C07K 15/00

(30) Priority: **04.03.91 US 663560**

(43) Date of publication of application:
**09.09.92 Bulletin 92/37**

(84) Designated Contracting States:
**DE FR GB NL**

(71) Applicant: **BOYCE THOMPSON INSTITUTE FOR PLANT RESEARCH, INC.**
**Tower Road**
**Ithaca, NY 14853-1801(US)**

(72) Inventor: **Granados, Robert R.**
**5 Eagleshead Road**
**Ithaca,New York 14859(US)**
Inventor: **Hashimoto, Yoshifumi, Depart. of Applied Biology**
**Kyoto Institute of Technology**
**Matsugasaki, Sakyo-ku, Kyoto 606(US)**

(74) Representative: **Buchner, Otto, Dr. et al**
**Patentanwälte Dipl.-Ing. Klaus Westphal Dr. rer. nat. Bernd Mussgnug Dr. rer. nat. Otto Buchner Flossmannstrasse 30a**
**W-8000 München 60(DE)**

(54) **Gene coded for a polypeptide which enhances virus infection of host insects.**

(57) A cloned gene coded for a polypeptide isolated from an occlusion body of a baculovirus such as Trichoplusia ni granulosis virus possessing a biological activity of enhancing virus infection of host insects by causing rapid degradation of the peritrophic membrane lining the midgut lumen of insects. Novel pesticides incorporating said gene are also claimed.

EP 0 502 236 A1

## FIELD OF THE INVENTION

The invention relates to the cloning and sequence of a novel virulence gene from a baculovirus for insect control. More particularly, the invention relates to the discovery of a DNA sequence and the deduced amino acid sequence for a polypeptide isolated from an occlusion body of a baculovirus and possessing a biological activity wherein said activity is the enhancement of the infectivity of certain viral pesticides.

The publications used to illuminate the background of the invention, and in particular cases, to provide additional details respecting its practice are incorporated herein by reference, and for convenience, are numerically referenced by the following text and respectively grouped in the appended bibliography. Copies of all of the references mentioned in this bibliography are attached to the **INFORMATION DISCLOSURE STATEMENT** filed with our parent application.

## BACKGROUND OF THE INVENTION

Present in the protein occlusion bodies (OBs) of some baculoviruses is a unique viral-encoded protein which enhances viral infection of the host insect. This protein has been referred to as the virus enhancing factor (VEF). Pest control compositions comprising this factor and nuclear polyhedrosis viruses are the subject matter of U.S. 4,973,667 and the copending application of one of the inventors, viz. Robert R. Granados, Serial No. 580,083 filed September 10, 1990 and entitled **BACULOVIRUS PROTEINS AND VIRAL PESTICIDES CONTAINING SAME**.

Studies on the mode of action of the VEF isolated from Trichoplusia ni granulosis virus (TnGV) showed that the VEF caused rapid degradation of the peritrophic membrane which lines the midgut lumen. Larval bioassays suggested that this alteration made the peritrophic membrane more permeable to invading baculoviruses resulting in at least a 25-fold increase in larval mortality (1, 2).

The closest prior art to the VEF protein of the present invention is believed to be a lipoprotein, originally isolated from a Hawaiian strain of Pseudaletia unipuncta granulosis virus (PuGV-H), and described by Tanada and co-workers (7, 8, 10) as the "synergistic factor" (SF). While SF enhances nuclear polyhedrosis virus infection in larvae (7), the mode of action of the SF and the viral enhancing protein (VEP) appear to be different since the SF is an absorption factor, involved in the attachment of virions to cells in vivo and in vitro (10). In addition, phospholipase C which inactivates SF does not inactivate the VEP, and depolymerization did not occur even after a 4 hour incubation of the VEP with phospholipase C with and without 1% sodium dodecyl sulfate.

Since the viral enhancing protein(s) are important at early stages of host infection, it is important to identify and locate the position of the VEF gene on the viral genome. A need, therefore, exists to clone and sequence the VEF gene of Trichoplusia ni (cabbage looper) granulosis virus (TnGV) DNA (DNA is an abbreviation of deoxyribonucleic acid) and to show sequence homology of the VEF virulence gene among different baculoviruses. It is an object of this invention to satisfy such a need.

## SUMMARY OF THE INVENTION

The above-mentioned object and other objects of the present invention which will hereinafter become more readily apparent from the following description have been attained by providing a VEF gene found in the granulin fraction of TnGV OBs purified by sephacryl column and comprising a DNA molecule encoding a polypeptide of molecular weight 104 kDa and possessing a biological activity wherein said polypeptide has 901 amino acid residues in total in the amino acid sequence of the polypeptide.

The gene encoding for the viral enhancing factor (VEF) of Trichoplusia ni granulosis virus has been cloned from a λgt11 expression library, and the complete nucleotide sequence determined. The VEF gene encodes a protein with a predicted molecular weight of 104 Kd which does not share homology to any previously reported proteins. The apparent promoter is located 4 bp upstream of the initiation codon and represents a consensus baculovirus late promoter (ATAAG). This has been confirmed by the identification of VEF mRNA in northern blots of infected larvae at 6 days but not 3 days post infection. Three repeats of the sequence 'TTACAAGA' which match the baculovirus late promoter in 4 of 5 nucleotides have been identified between 149 and 192 bp upstream of the initiation codon. While the function of these sequences is unknown, they are not believed to be transcriptionally active since they diverge from the consensus promoter at the invariant 'T' position. Using the VEF gene as a probe in southern blots of genomic DNAs, homologous sequences have been identified in Pseudaletia unipuncta granulosis virus - Hawaiian strain (PnGV-H) and Heliothis armigera GV (HaGV) but not Erinnyis ello GV, (EeGV), Autographa californica nuclear polyhedrosis virus (AcMNPV) or Trichoplusia ni nuclear polyhedrosis virus (TnSNPV). In addition, SDS-PAGE analysis of dissolved viral occlusion bodies have demonstrated proteins with a molecular weight similar to VEF in PuGV-H and HaGV.

## BRIEF DESCRIPTION OF THE DRAWINGS

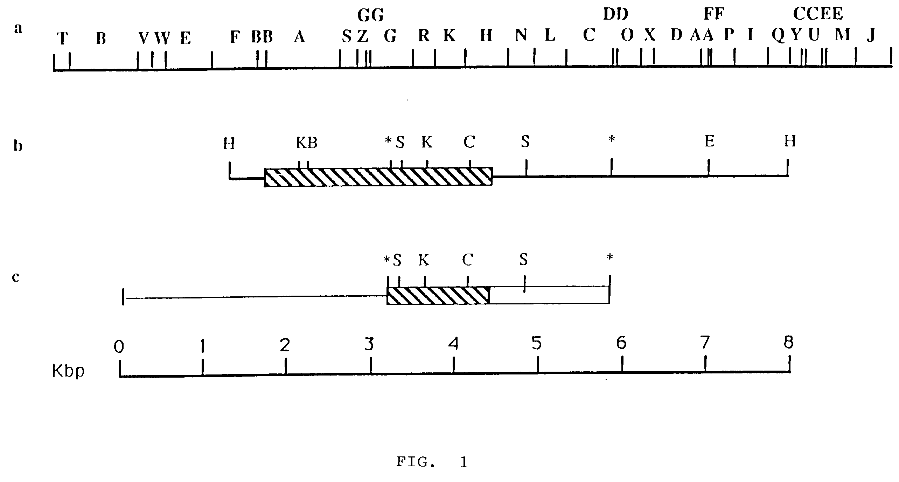

A more complete appreciation of the invention and the attendant advantages thereof will be readily attained as the same becomes better understood by reference to the following details of description when considered in connection with the accompanying drawings, i.e. Figures 1-6.

Fig. 1 Mapping of the VEF gene of TnGV. a) a Hind III restruction map of the TnGV genome. By convention, the smallest fragment containing all of the granulin gene is assigned to be the first fragment at the left of the linearized map. A fine map of the b) Hind III-M fragment of TnGV and c) fusion gene of Lambda F. The striped box indicates the position of the VEF gene while the open box indicates non-coding TnGV sequences inserted into the λgt11 vector. The entire insert in lambda F is demarkated by the asterisks. The size of the DNA is indicated by scale, and the restriction sites for BamHI(B), ClaI(C), EcoRI-(E), HindIII(H), KpnI(K), and SalI(S) are indicated.

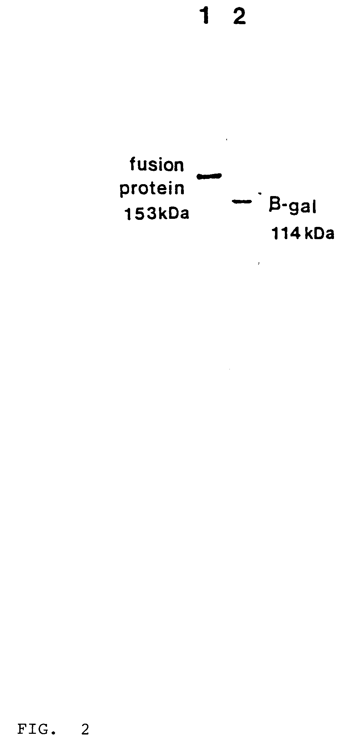

Fig. 2 Western blot analysis of lambda lysogens form lambda F (lane 1 or λgt11 (lane 2) probed with either anti-VEF polyclonal antibody (lane 1) or an anti-$\beta$-galactosidase monoclonal antibody (lane 2). Lysogens were first separated on a 10% SDS-PAGE gel and then electrophoretically transferred to nitrocellulose. The 153K protein identified by the anti-VEF polyclonal antibody consists of 39K VEF carbocy-terminal and 114K of $\beta$-galactosidase.

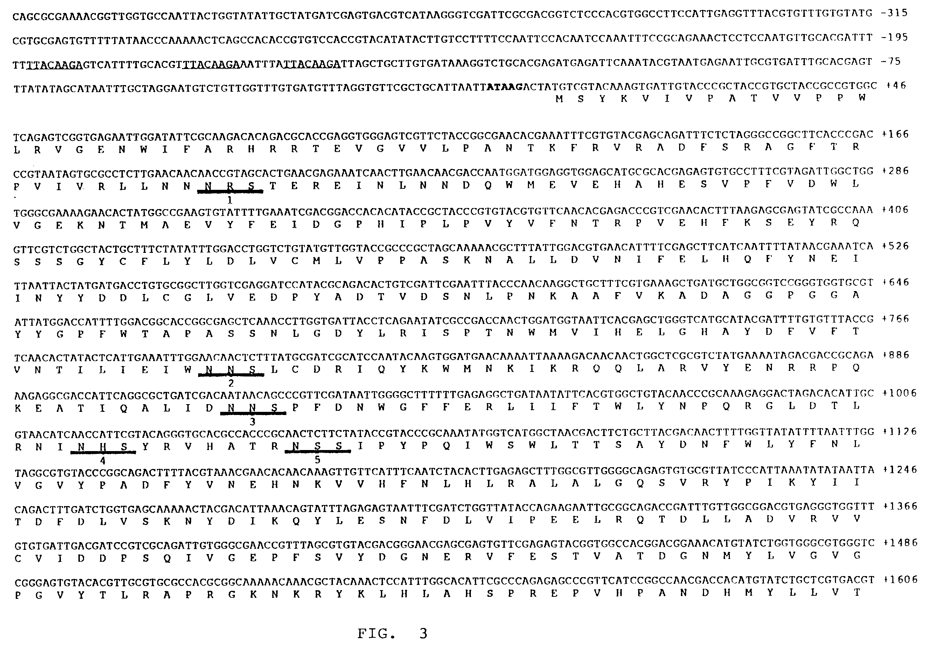

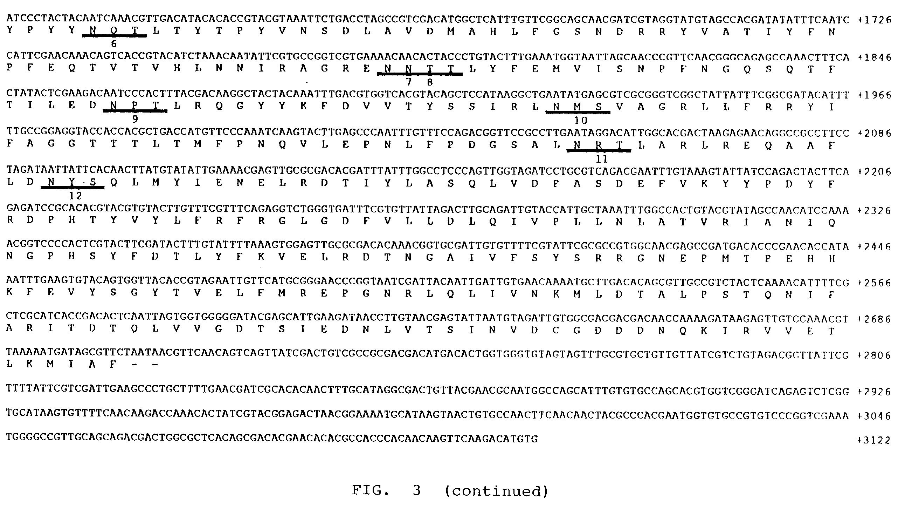

Fig. 3 The nucleotide sequence of the VEF gene from TnGV. The gene has been translated using the single-letter amino acid code. The bolded sequence represents the consensus baculovirus late promoter (5), and the underlined sequences represents 3 repeats of the sequence (ACAAG) which matches the promoter in 4 of the 5 base pairs. Double underlined sequences indicate possible glycosylation sites. The DNA sequence of a 3.5 Kb portion of Hind III-M fragment was determined by dideoxy chain termination method using bacteriophage T7 DNA polymerase. Sequence data were compiled and analyzed using the software program of PCGENE. A 14 mer consensus sequency (-169 to -155) commonly present in a transcription starting area in baculovirus genes expressed late in infection (5) and a sequence indicating a strong $\beta$-turn probability (+628 to +639) are also underlined. In this sequence, A stands for deoxyadenyl, G for deoxyguanyl, C deoxycytidyl, and T is thymidyl. The amino acids encoded by the above DNA are designated below the appropriate nucleotide triplet. Accordingly, MET is methionine; LYS is lysine; PRO is proline; GLU is glutamate, LEU is leucine; THR is threonine; ALA is alanine; SER is serine; VAL is valine; PHE is phenylalanine; ILE is isoleucine; GLY is glycine; ASP is aspartic acid; GLN is glutamine; ARG is arginine; CYS is cysteine; TRP is tryptophan; ASN is asparagine; HIS is histidine; and TYR is tyrosine.

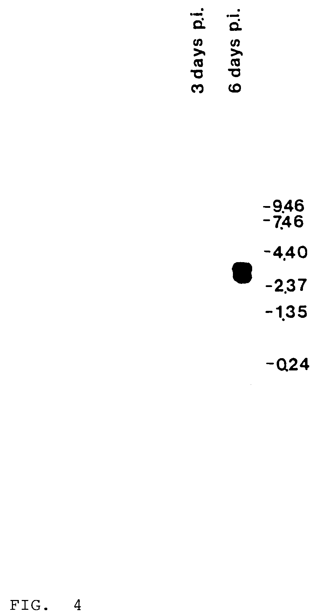

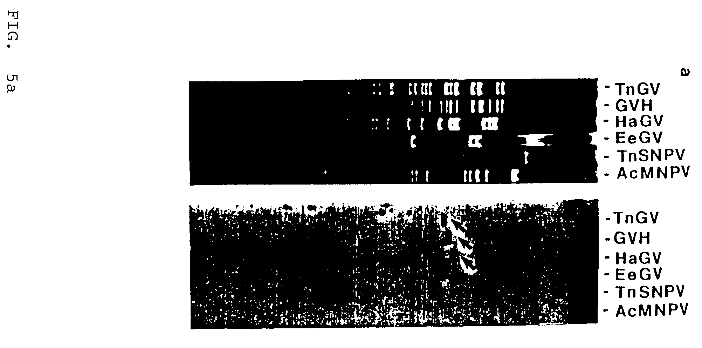

Fig. 4 Northern blot of total RNA isolated from infected larvae. Total RNA was isolated from T. ni larvae at 3 and 6 days PI with TnGV. Ten micrograms of RNA were electrophoresed in a denaturing 1.5% agarose and northern blotted following the methods of Dwyer and Granados, Journal of Virology 62 (5), 1535-1542. Blots were probed with the internal KpnI fragment of TnGV-VEF gene under high stringency conditions. No hybridization was found to RNA isolated at 3 days PI. However, 2 RNA species of 2.7 and 3.3. Kbp hydrbidized at 6 days PI. This indicated that the VEF gene was probably a late gene.

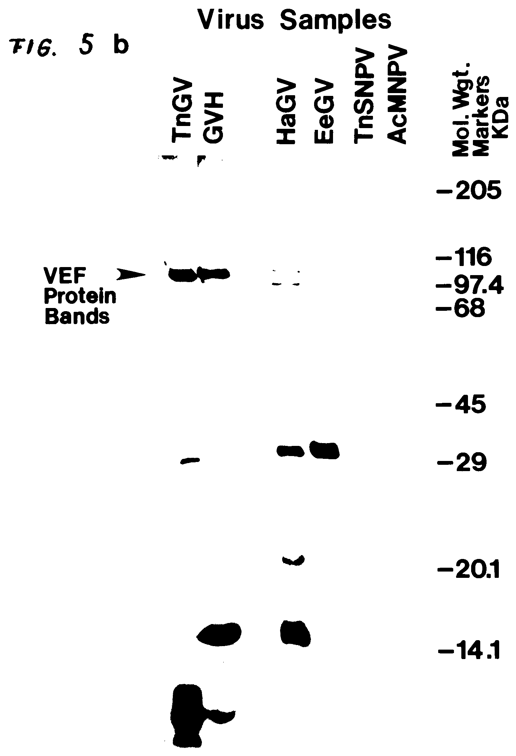

Fig. 5 Southern hybridization and SDS-PAGE analysis of TnGV and 5 other baculoviruses. a) Genomic baculovirus was digested with HindIII and electrophoresed on a 0.75% agarose gel. The DNA was transferred to nitrocellulose and probed with the internal KpNI fragment of TnGV-VEF and washed under high stringency conditions. Homologous sequences were identified in Pseudaletia unipuncta GV Hawaiian strain (PuGV-H), and Heliothis armigera GV (HaGV). b) Occlusion bodies are dissolved in 0.05 M NaCO pH 10.5 for 15 minutes at room temperature and the nucleocapsids pelleted by centrifugation at 14,000 xg. The supernatants were removed and electrophorsed in a 10% SDS-PAGE gel and stained with coomassie blue.

## DETAILED DESCRIPTION OF THE PREFERRED EMBODIMENT

The VEF present in the granulin fraction of TnGV OBs was purified in the following manner:

1.7 x 10-12 TnGV OBs were dissolved in 1 ml 0.05 M $Na_2$ $CO_3$ for 15 min. at room temperature, and layered on a 20% sucrose cushion in $H_2$ O and centrifuged for 45 min. at 126,000 g at 4°C. The granulin fraction remained on top of the sucrose cushion and was collected. After an incubation of 5 hrs at 28°C, the granulin fraction was applied onto a Sephacryl-S-200 column (2.6 x 34 cm) and eluted with 50 mM Tris-HC1 pH 7.0, 0.1 M NaCl at 1.5 ml/min, and the absorption of the eluate measured at 280 nm. The first peak containing VEF protein was pooled and used for experiment. D, dead larvae; P, healthy pupa.

Following purification by means of the sephacryl column, the biological activity of the protein was determined by larval bioassay. As little as 40 ng of VEF, in addition to the virus inoculum, per larva was sufficient to increase the infection of fifth instar T. ni larvae from 17% to 95%.

A cloning and expression vector, λgtll, was used for construction of genomic library of TnGV and for isolation of the VEF gene (3). Antibodies were raised against Sephacryl-column purified VEF from granulin fraction after alkali solubilization of OBs (1) and were used for immunoblotting to screen for positive clones. Through several steps of screening approximately 6000 plagues, a clone was selected containing the longest viral DNA insert, λVEF-F (Fig. 1). Southern blot hybridization analysis of TnGV DNA Hind III digests, probed with the λVEF-F clone insert, revealed that the VEF gene existed on the Hind III-M fragment. Western blot analysis of the fusion protein expressed in lysogenic E. coli (Y1089 strain) transfected with λVEF-F had a molecular weight of 153 kDa. This suggested a fusion protein gene consisting of 39kDa of the VEF carboxy terminal end and the 114 kDa beta-gal gene. Since the VEF has a size of 101 kDa (1), the position of the VEF gene on a fine map of the Hind III-M fragment was predicted and a 3.5 kbp DNA portion was sequenced (Fig. 1).

Sequence analysis showed an open reading frame of 2.7 kbp DNA corresponding to the size of the VEF polypeptide at the predicted location of the VEF gene (Fig. 3). The deduced size of the polypeptide was 101,200 daltons and consisted of 901 amino acid residues. There are no sites for lipophilic modification (Lys/X/X/Cys/X/X/Asn). To determine the presence of the VEF gene among several isolates of baculoviruse a 1.5 kbp portion of the VEF gene was probed onto a Southern blot of different virus DNA fragments digested with Hind III restriction enzyme under high stringency condition (12). The result showed that two granulosis virus DNAs, isolated from P. unipuncta-Hawaiian strain (GVH) and Heliothis armigera (HaGV), contained a sequence homologous to the TnGV VEF gene (Fig. 4). DNA isolated from Erinnyis ello (EeGV) did not contain sequences homologous to the VEF gene probe. The restriction enzyme digestion pattern of DNA from TnGV, GVH, and HaGV were very similar, whereas EeGV exhibited a very distinct DNA Profile. The probe did not hydridize with DNAs from two nuclear polyhedrosis viruses (Fig. 4). Temporal gene expression of the VEF gene was examined by Northern blot analysis of total RNA from TnGV-infected T. ni larvae at 3 and 6 days p.i. A probe with a size of 1.5 Kb KpnI-V fragment, a part of the VEF gene, showed no hybridization with RNAs at 3 days p.i. but showed strong hybridization with two RNA species with sizes of 2.7 Kb and 3.3. Kb at 6 days p.i. (Fig. 5a). The TnGV-VEF present in the granulin fraction of alkaline dissolved OBs was resolved as a 101 kDa protein on a SDS-polyacrylamide gel. To determine the presence of high molecular weight polypeptides in granulin or polyhedrin fractions from six baculoviruses, these virus samples were analyzed by SDS-PAGE (Fig 5a). In the granulin fractions from TnGV, GVH and HaGV, polypeptides with a size of 101 KDa, 103 kDa, and a complex of 104 kDa and 94 kDa were detected, respectively. The single high molecular weight polypeptide (103 kDa) from GVH appears to be analogous to the 101 kDa protein from TnGV (Fig. 5a and Y. Tanada, personal communication). The assignment of a VEF function to either the 94 or 104 kDa polypeptides from HaGV is not clear at this time. No polypeptides with a size of approximately 100 kDa were present in EeGV, TnSNPV, and AcMNPV. Three of the GVs examined, TnGV, GVH, and HaGV all infect the noctuid species T. ni, whereas EeGV grows only in the sphyngid species, E. ello.

## SUMMARY OF RESULTS

Two positive clones were identified from the approximately 6000 plagues screened with a α-VEF polyclonal antiserum. Both clones had identical inserts of 2.8 Kb mapped to the HindIII-M fragment of the TnGV genome (92.2 to 95.8 MV; Fig 1a). Other TnGV fragments hybridizing to the clones included the 6.7 Kb EcoRI-K and the BamHI-FI6 doublet. Detailed maps of both TnGV HindIII-M and the insert DNA were generated using several restriction enzymes (Fig. 1b, c).

Western blot analysis using both an anti-VEF polyclonal antisera and an anti-β-galactosidase monoclonal antibody (Promega, Madison, WI) demonstrated that the fusion protein generated by Lambda-F had an M of 153 Kd which presumably consisted of 39 Kd of VEF carboxy-terminal and 114 Kd of β-galactosidase protein (Fig. 2). The VEF reading frame.

Five additional baculoviruses were tested for the presence of VEF-homologous proteins by both DNA hybridization and SDS-PAGE analysis of dissolved occlusion bodies (Fig. 4A & B). Hind-III genomic digest of the 5 baculovirus DNAs under low stringency conditions, using a restriction fragment from with the VEF ORF as a probe, showed homology between TnGV and 2 other granulosis viruses {PuGV-H and Heliothis armigera GV (HaGV)}. No apparent homology was seen to either Erinnyis ello GV (EeGV), Trichoplusia ni singly enveloped NPV (TnSNPV), or Autographa californica MNPV (AcMNPV) (Fig. 5A).

The coded gene of the present invention can be used in engineering new viral pesticides with enhanced efficacy. For example, it can be used alone as a biopesticide or in combination with known biological insecticides such as BT or with synthetic chemical insecticides. The gene product of this invention can also be used to produce VEF in any microbial production system, e.g. E. coli., bacillus or streptococcus. It can be introduced into a variety of hosts such as plants for protection against insects or microbes as biological active agents.

In addition, VEF can be engineered to be expressed in transgenic plants. As insects feed on these plants, they would ingest a constant does of VEF. While the exact effect of this on the insect is undetermined, it can be hypothesized that prolonged disruption of the peritrophic membrane (PM) may allow opportunistic microbes to infect and kill the insects. Also, VEF may increase the efficiency of other biopesticides (i.e. Bt delta endotoxin) by removing a major mechanical barrier--the PM.

The coded gene of the present invention has been found to play a significant role as a determinant of virulence at the initial stage of infection in insect hosts. Knowledge gained in cloning and sequencing the VEF gene should prove useful in helping to unravel the mechanism(s) of enhanced virus infection by enhancement factors present within the occlusion body matrix.

Notwithstanding that reference has been made to particular preferred embodiments, it will be understood that the present invention is not to be construed as limited as such, but rather to the lawful scope of the appended claims. In other words, the subject invention includes not only the specific nucleotide sequences depicted herein, but also all equivalent nucleotide sequences coding for molecules with substantially the same biological activity of enhancing the infectivity of baculoviruses. The term "equivalent" is being used in ordinary patent usage here as denoting a nucleotide sequence which performs substantially as the nucleotide sequence identified herein to produce molecules with substantially the same biological activity in essentially the same kind of hosts. Within this definition are subfragments which have biological activity of enhancing the infectivity of baculoviruses.

Inasmuch as the protein, i.e., the gene product, of the present invention has been defined by means of deductive amino acid sequencing, c.f. Fig. 3, it is to be understood that for this particular protein, embraced herein, natural allelic variations exist and occur from individual to individual. These variations may be demonstrated by (an) amino acid difference(s) in the overall sequence or by deletions, substitutions, insertions, inversions or additions of (an) amino acid(s) in said sequence. All such alletic variations are included within the scope of the present invention.

## BIBLIOGRAPHY

1. Derksen, A.G.S. and Granados, R.R. Virology. 167: 242-25- (1981).
2. Peters, W. and Wiese, B. J. Insect Physio. 32:43-49 (1986).
3. Synder, M., Sweetsen, D., Young, R.A. and Davis, R.W. Meth. Enzym. 154:107-128 (1985).
4. Vlak, J.M. and Smith, G.E. J. Virol. 41:1118-1121 (1981).
5. Rohrmann, G.F. J. Gen. Virol. 67:1499-1513 (1986).
6. Tanada, V. and Hukuhara, T. J. Invertebr. Pathol. 17:116-126 (1971).
7. Tanada, Y. and Hara, S. Nature. 254:328-329 (1975).
8. Tanada, T. Insect Pathol. 1:215-231 (1959).
9. Tanada, Y. Inoue, H., Hess, R.T., and Omi, E.M. J. Inverebr. Pathol. 34:249-255 (1980).
10. Tanada, Y. J. Invertebr. Pathol. 45:125-138 (1985).
11. Yamamoto, T., Kita, H. and Tanada, Y. J. Gen. Virol. 45:371-381 (1979).
12. Maniatis, T., Fritsch, E.F. and Sambrook, J. Molecular Cloning: A Laboratory Manual. Cold Spring Harbor Laboratory, Cold Spring Harbor, N.Y. (1982).
13. Hamm, J.J. and Paschke, J.D. J. Insect Pathol. 5:187-197 (1963).
14. Whitlock, V.H. J. Invertebr. Pathol. 23:70-75 (1974).
15. Granados, R.R. and Williams, K.A. The Biology of Baculoviruses, CRC Press, Fl. Vol I:89-108 (1986).
16. Miller, L.K. The Biology of Baculoviruses, CRC Press, Fl. Vol I:217-238 (1986).

SEQUENCE ID NUMBER: 1
SEQUENCE TYPE: Nucleotide with corresponding protein
SEQUENCE LENGTH: 3556 base pairs

STRANDEDNESS: Single-stranded
TOPOLOGY: Linear
MOLECULE TYPE: genomic DNA

ORIGINAL SOURCE
ORGANISM: Insect Baculovirus
VIRUS TYPE: *Trichoplusia ni* Granulosis Virus
IMMEDIATE EXPERIMENTAL SOURCE: Clone of genomic DNA into plasmid
pACYC184

FEATURES
from 434 to 3140 bp mature protein
from 427 to 432 bp baculovirus late promoter
from 65 to 67 aa possible glycosylation site 1
from 265 to 267 aa possible glycosylation site 2
from 306 to 308 aa possible glycosylation site 3
from 339 to 341 aa possible glycosylation site 4
from 349 to 351 aa possible glycosylation site 5
from 540 to 542 aa possible glycosylation site 6
from 594 to 596 aa possible glycosylation site 7
from 595 to 597 aa possible glycosylation site 8
from 621 to 623 aa possible glycosylation site 9
from 642 to 644 aa possible glycosylation site 10
from 683 to 685 aa possible glycosylation site 11
from 698 to 700 aa possible glycosylation site 12
IDENTIFICATION: Experimental

PROPERTIES: Viral Enhancing Protein
BIOLOGICAL/ENZYMATIC ACTIVITY: degrades specific peritrophic membrane
proteins
SUBCELLULAR LOCALIZATION: in the mature occlusion body of the virus


```
CAGCGCGAAA ACGGTTGGTG CCAATTACTG GTATATTGCT ATGATCGAGT GACGTCATAA        60
GGGTCGATTC GCGACGGTCT CCCACGTGGC CTTCCATTGA GGTTTACGTG TTTGTGTATG       120
CGTGCGAGTG TTTTTATAAC CCAAAAACTC AGCCACACCG TGTCCACCGT ACATATACTT       180
GTCCTTTTCC AATTCCACAA TCCAAATTTC CGCAGAAACT CCTCCAATGT TGCACGATTT       240
TTTTACAAGA GTCATTTTGC ACGTTTACAA GAAATTTATT ACAAGATTAG CTGCTTGTGA       300
TAAAGGTCTG CACGAGATGA GATTCAAATA CGTAATGAGA ATTGCGTGAT TTGCACGAGT       360
TTATATAGCA TAATTTGCTA GGAATGTCTG TTGGTTTGTG ATGTTTAGGT GTTCGCTGCA       420
TTAATTATAA GACT ATG TCG TAC AAA GTG ATT GTA CCC GCT ACC GTG CTA       470
               MET Ser Tyr Lys Val Ile Val Pro Ala Thr Val Leu
                1           5                    10

CCG CCG TGG CTC AGA GTC GGT GAG AAT TGG ATA TTC GCA AGA CAC AGA       518
Pro Pro Trp Leu Arg Val Gly Glu Asn Trp Ile Phe Ala Arg His Arg
         15             20                25
```

```
CGC ACC GAG GTG GGA GTC GTT CTA CCG GCG AAC ACG AAA TTT CGT GTA        566
Arg Thr Glu Val Gly Val Val Leu Pro Ala Asn Thr Lys Phe Arg Val
    30                  35                  40

CGA GCA GAT TTC TCT AGG GCC GGC TTC ACC CGA CCC GTA ATA GTG CGC        614
Arg Ala Asp Phe Ser Arg Ala Gly Phe Thr Arg Pro Val Ile Val Arg
    45                  50                  55                  60

CTC TTG AAC AAC AAC CGT AGC ACT GAA CGA GAA ATC AAC TTG AAC AAC        662
Leu Leu Asn Asn Asn Arg Ser Thr Glu Arg Glu Ile Asn Leu Asn Asn
                    65                  70                  75

GAC CAA TGG ATG GAG GTG GAG CAT GCG CAC GAG AGT GTG CCT TTC GTA        710
Asp Gln Trp MET Glu Val Glu His Ala His Glu Ser Val Pro Phe Val
                80                  85                  90

GAT TGG CTG GTG GGC GAA AAG AAC ACT ATG GCC GAA GTG TAT TTT GAA        758
Asp Trp Leu Val Gly Glu Lys Asn Thr MET Ala Glu Val Tyr Phe Glu
            95                  100                 105

ATC GAC GGA CCA CAC ATA CCG CTA CCC GTG TAC GTG TTC AAC ACG AGA        806
Ile Asp Gly Pro His Ile Pro Leu Pro Val Tyr Val Phe Asn Thr Arg
            110                 115                 120

CCC GTC GAA CAC TTT AAG AGC GAG TAT CGC CAA AGT TCG TCT GGC TAC        854
Pro Val Glu His Phe Lys Ser Glu Tyr Arg Gln Ser Ser Ser Gly Tyr
125                 130                 135                 140

TGC TTT CTA TAT TTG GAC CTG GTC TGT ATG TTG GTA CCG CCC GCT AGC        902
Cys Phe Leu Tyr Leu Asp Leu Val Cys MET Leu Val Pro Pro Ala Ser
                145                 150                 155

AAA AAC GCT TTA TTG GAC GTG AAC ATT TTC GAG CTT CAT CAA TTT TAT        950
Lys Asn Ala Leu Leu Asp Val Asn Ile Phe Glu Leu His Gln Phe Tyr
                160                 165                 170

AAC GAA ATC ATT AAT TAC TAT GAT GAC CTG TGC GGC TTG GTC GAG GAT        998
Asn Glu Ile Ile Asn Tyr Tyr Asp Asp Leu Cys Gly Leu Val Glu Asp
            175                 180                 185

CCA TAC GCA GAC ACT GTC GAT TCG AAT TTA CCC AAC AAG GCT GCT TTC       1046
Pro Tyr Ala Asp Thr Val Asp Ser Asn Leu Pro Asn Lys Ala Ala Phe
            190                 195                 200

GTG AAA GCT GAT GCT GGC GGT CCG GGT GGT GCG TAT TAT GGA CCA TTT       1094
Val Lys Ala Asp Ala Gly Gly Pro Gly Gly Ala Tyr Tyr Gly Pro Phe
205                 210                 215                 220

TGG ACG GCA CCG GCG AGC TCA AAC CTT GGT GAT TAC CTC AGA ATA TCG       1142
Trp Thr Ala Pro Ala Ser Ser Asn Leu Gly Asp Tyr Leu Arg Ile Ser
                225                 230                 235
```

```
CCG ACC AAC TGG ATG GTA ATT CAC GAG CTG GGT CAT GCA TAC GAT TTT    1190
Pro Thr Asn Trp MET Val Ile His Glu Leu Gly His Ala Tyr Asp Phe
            240             245             250

GTG TTT ACC GTC AAC ACT ATA CTC ATT GAA ATT TGG AAC AAC TCT TTA    1238
Val Phe Thr Val Asn Thr Ile Leu Ile Glu Ile Trp Asn Asn Ser Leu
            255             260             265

TGC GAT CGC ATC CAA TAC AAG TGG ATG AAC AAA ATT AAA AGA CAA CAA    1286
Cys Asp Arg Ile Gln Tyr Lys Trp MET Asn Lys Ile Lys Arg Gln Gln
            270             275             280

CTG GCT CGC GTC TAT GAA AAT AGA CGA CCG CAG AAA GAG GCG ACC ATT    1334
Leu Ala Arg Val Tyr Glu Asn Arg Arg Pro Gln Lys Glu Ala Thr Ile
285             290             295             300

CAG GCG CTG ATC GAC AAT AAC AGC CCG TTC GAT AAT TGG GGC TTT TTT    1382
Gln Ala Leu Ile Asp Asn Asn Ser Pro Phe Asp Asn Trp Gly Phe Phe
            305             310             315

GAG AGG CTG ATA ATA TTC ACG TGG CTG TAC AAC CCG CAA AGA GGA CTA    1430
Glu Arg Leu Ile Ile Phe Thr Trp Leu Tyr Asn Pro Gln Arg Gly Leu
            320             325             330

GAC ACA TTG CGT AAC ATC AAC CAT TCG TAC AGG GTG CAC GCC ACC CGC    1478
Asp Thr Leu Arg Asn Ile Asn His Ser Tyr Arg Val His Ala Thr Arg
            335             340             345

AAC TCT TCT ATA CCG TAC CCG CAA ATA TGG TCA TGG CTA ACG ACT TCT    1526
Asn Ser Ser Ile Pro Tyr Pro Gln Ile Trp Ser Trp Leu Thr Thr Ser
            350             355             360

GCT TAC GAC AAC TTT TGG TTA TAT TTT AAT TTG GTA GGC GTG TAC CCG    1574
Ala Tyr Asp Asn Phe Trp Leu Tyr Phe Asn Leu Val Gly Val Tyr Pro
365             370             375             380

GCA GAC TTT TAC GTA AAC GAA CAC AAC AAA GTT GTT CAT TTC AAT CTA    1622
Ala Asp Phe Tyr Val Asn Glu His Asn Lys Val Val His Phe Asn Leu
            385             390             395

CAC TTG AGA GCT TTG GCG TTG GGG CAG AGT GTG CGT TAT CCC ATT AAA    1670
His Leu Arg Ala Leu Ala Leu Gly Gln Ser Val Arg Tyr Pro Ile Lys
            400             405             410

TAT ATA ATT ACA GAC TTT GAT CTG GTG AGC AAA AAC TAC GAC ATT AAA    1718
Tyr Ile Ile Thr Asp Phe Asp Leu Val Ser Lys Asn Tyr Asp Ile Lys
            415             420             425

CAG TAT TTA GAG AGT AAT TTC GAT CTG GTT ATA CCA GAA GAA TTG CGG    1766
Gln Tyr Leu Glu Ser Asn Phe Asp Leu Val Ile Pro Glu Glu Leu Arg
            430             435             440
```

```
CAG ACC GAT TTG TTG GCG GAC GTG AGG GTG GTT TGT GTG ATT GAC GAT    1814
Gln Thr Asp Leu Leu Ala Asp Val Arg Val Val Cys Val Ile Asp Asp
445             450             455             560

CCG TCG CAG ATT GTG GGC GAA CCG TTT AGC GTG TAC GAC GGG AAC GAG    1862
Pro Ser Gln Ile Val Gly Glu Pro Phe Ser Val Tyr Asp Gly Asn Glu
                465             470             475

CGA GTG TTC GAG AGT ACG GTG GCC ACG GAC GGA AAC ATG TAT CTG GTG    1910
Arg Val Phe Glu Ser Thr Val Ala Thr Asp Gly Asn MET Tyr Leu Val
                480             485             490

GGC GTG GGT CCG GGA GTG TAC ACG TTG CGT GCG CCA CGC GGC AAA AAC    1958
Gly Val Gly Pro Gly Val Tyr Thr Leu Arg Ala Pro Arg Gly Lys Asn
            495             500             505

AAA CGC TAC AAA CTC CAT TTG GCA CAT TCG CCC AGA GAG CCC GTT CAT    2006
Lys Arg Tyr Lys Leu His Leu Ala His Ser Pro Arg Glu Pro Val His
        510             515             520

CCG GCC AAC GAC CAC ATG TAT CTG CTC GTG ACG TAT CCC TAC TAC AAT    2054
Pro Ala Asn Asp His MET Tyr Leu Leu Val Thr Tyr Pro Tyr Tyr Asn
525             530             535             540

CAA ACG TTG ACA TAC ACA CCG TAC GTA AAT TCT GAC CTA GCC GTC GAC    2102
Gln Thr Leu Thr Tyr Thr Pro Tyr Val Asn Ser Asp Leu Ala Val Asp
                545             550             555

ATG GCT CAT TTG TTC GGC AGC AAC GAT CGT AGG TAT GTA GCC ACG ATA    2150
MET Ala His Leu Phe Gly Ser Asn Asp Arg Arg Tyr Val Ala Thr Ile
            560             565             570

TAT TTC AAT CCA TTC GAA CAA ACA GTC ACC GTA CAT CTA AAC AAT ATT    2198
Tyr Phe Asn Pro Phe Glu Gln Thr Val Thr Val His Leu Asn Asn Ile
            575             580             585

CGT GCC GGT CGT GAA AAC AAC ACT ACC CTG TAC TTT GAA ATG GTA ATT    2246
Arg Ala Gly Arg Glu Asn Asn Thr Thr Leu Tyr Phe Glu MET Val Ile
            590             595             600

AGC AAC CCG TTC AAC GGG CAG AGC CAA ACT TTC ACT ATA CTC GAA GAC    2294
Ser Asn Pro Phe Asn Gly Gln Ser Gln Thr Phe Thr Ile Leu Glu Asp
605             610             615             620

AAT CCC ACT TTA CGA CAA GGC TAC TAC AAA TTT GAC GTG GTC ACG TAC    2342
Asn Pro Thr Leu Arg Gln Gly Tyr Tyr Lys Phe Asp Val Val Thr Tyr
                625             630             635

AGC TCC ATA AGG CTG AAT ATG AGC GTC GCG GGT CGG CTA TTA TTT CGG    2390
Ser Ser Ile Arg Leu Asn MET Ser Val Ala Gly Arg Leu Leu Phe Arg
            640             645             650
```

```
CGA TAC ATT TTT GCC GGA GGT ACC ACC ACG CTG ACC ATG TTC CCA AAT      2438
Arg Tyr Ile Phe Ala Gly Gly Thr Thr Thr Leu Thr MET Phe Pro Asn
        655                 660                 665

CAA GTA CTT GAG CCC AAT TTG TTT CCA GAC GGT TCC GCC TTG AAT AGG      2486
Gln Val Leu Glu Pro Asn Leu Phe Pro Asp Gly Ser Ala Leu Asn Arg
        670                 675                 680

ACA TTG GCA CGA CTA AGA GAA CAG GCC GCC TTC CTA GAT AAT TAT TCA      2534
Thr Leu Ala Arg Leu Arg Glu Gln Ala Ala Phe Leu Asp Asn Tyr Ser
685                 690                 695                 700

CAA CTT ATG TAT ATT GAA AAC GAG TTG CGC GAC ACG ATT TAT TTG GCC      2582
Gln Leu MET Tyr Ile Glu Asn Glu Leu Arg Asp Thr Ile Tyr Leu Ala
                705                 710                 715

TCC CAG TTG GTA GAT CCT GCG TCA GAC GAA TTT GTA AAG TAT TAT CCA      2630
Ser Gln Leu Val Asp Pro Ala Ser Asp Glu Phe Val Lys Tyr Tyr Pro
                720                 725                 730

GAC TAC TTC AGA GAT CCG CAC ACG TAC GTG TAC TTG TTT CGT TTC AGA      2678
Asp Tyr Phe Arg Asp Pro His Thr Tyr Val Tyr Leu Phe Arg Phe Arg
        720                 740             -       745

GGT CTG GGT GAT TTC GTG TTA TTA GAC TTG CAG ATT GTA CCA TTG CTA      2726
Gly Leu Gly Asp Phe Val Leu Leu Asp Leu Gln Ile Val Pro Leu Leu
        750                 755                 760

AAT TTG GCC ACT GTA CGT ATA GCC AAC ATC CAA AAC GGT CCC CAC TCG      2774
Asn Leu Ala Thr Val Arg Ile Ala Asn Ile Gln Asn Gly Pro His Ser
765                 770                 775                 780

TAC TTC GAT ACT TTG TAT TTT AAA GTG GAG TTG CGC GAC ACA AAC GGT      2822
Tyr Phe Asp Thr Leu Tyr Phe Lys Val Glu Leu Arg Asp Thr Asn Gly
                785                 790                 795

GCG ATT GTG TTT TCG TAT TCG CGC CGT GGC AAC GAG CCG ATG ACA CCC      2870
Ala Ile Val Phe Ser Tyr Ser Arg Arg Gly Asn Glu Pro MET Thr Pro
                800                 805                 810

GAA CAC CAT AAA TTT GAA GTG TAC AGT GGT TAC ACC GTA GAA TTG TTC      2918
Glu His His Lys Phe Glu Val Tyr Ser Gly Tyr Thr Val Glu Leu Phe
                815                 820                 825

ATG CGG GAA CCC GGT AAT CGA TTA CAA TTG ATT GTG AAC AAA ATG CTT      2966
MET Arg Glu Pro Gly Asn Arg Leu Gln Leu Ile Val Asn Lys MET Leu
        830                 835                 840

GAC ACA GCG TTG CCG TCT ACT CAA AAC ATT TTC GCT CGC ATC ACC GAC      3014
Asp Thr Ala Leu Pro Ser Thr Gln Asn Ile Phe Ala Arg Ile Thr Asp
845                 850                 855                 860
```

```
ACT CAA TTA GTG GTG GGG GAT ACG AGC ATT GAA GAT AAC CTT GTA ACG    3062
Thr Gln Leu Val Val Gly Asp Thr Ser Ile Glu Asp Asn Leu Val Thr
                865                     870                 875

AGT ATT AAT GTA GAT TGT GGC GAC GAC GAC AAC CAA AAG ATA AGA GTT    3110
Ser Ile Asn Val Asp Cys Gly Asp Asp Asp Asn Gln Lys Ile Arg Val
            880                     885                 890

GTG GAA ACG TTA AAA ATG ATA GCG TTC TAA TAACGTTCAA CAGTCAGTTA      3160
Val Glu Thr Leu Lys MET Ile Ala Phe
        895                     900

TCGACTGTCG CCGCGACGAC ATGACACTGG TGGGTGTAGT AGTTTGCGTG CTGTTGTTAT  3220
CGTCTGTAGA CGGTTATTCG TTTTATTCGT CGATTGAAGC CCTGCTTTTG AACGATCGCA  3280
CACAACTTTG CATAGGCGAC TGTTACGAAC GCAATGGCCA GCATTTGTGT GCCAGCACGT  3340
GGTCGGGATC AGAGTCTCGG TGCATAAGTG TTTTCAACAA GACCAAACAC TATCGTACGG  3400
AGACTAACGG AAAATGCATA AGTAACTGTG CCAACTTCAA CAACTACGCC CACGAATGGT  3460
GTGCCGTGTC CCGGTCGAAA TGGGGCCGTT GCAGCAGACG ACTGGCGCTC ACAGCGACAC  3520
GAACACACGC CACCCACAAC AAGTTCAAGA CATGTG                            3556
```

## Claims

1. A cloned gene derived from a granulosis virus comprising a DNA sequence of nucleotides encoding a polypeptide possessing a biological activity wherein said activity is the enhancement of infectivity of baculoviruses wherein said polypeptide has been 901 amino acid residues in total in the amino acid sequence of the polypeptide.

2. The gene of Claim 1 comprising a DNA sequence of nucleotides as shown in Fig. 3 and which encodes a polypeptide having the amino acid sequence of Fig. 3.

3. A method for improving the efficacy of viral pesticides which comprises treating the pest with a viral composition containing the VEF gene of Claim 1.

4. An insecticide comprising a polypeptide characterized by having the amino acid sequence of Fig. 3.

5. A biopesticide comprising the gene product of Claim 1.

6. A composition comprising the gene product of Claim 1 and a pesticide.

7. A toxicant composition comprising the gene product of Claim 1 and a biological insecticide.

8. A toxicant composition comprising the gene product of Claim 1 and a synthetic chemical insecticide.

9. A microbial or cellular protein system for the production of the viral enhancing factor (VEF) which includes the gene product of Claim 1.

10. The microbial system of Claim 9 in which the microorganisms are selected from the group consisting of E. coli, bacillus, and streptococcus.

11. Process of introducing the gene product of Claim 1 into plants for protection against insects.

12. Process of introducing the gene product of Claim 1 into microbes useful as biologically active agents.

13. A cloned gene comprising a DNA sequence of base pairs encoding a polypeptide possessing a biological activity wherein said activity is the enhancement of infectivity of baculoviruses and wherein the polypeptide amino acid sequence differs from that exhibited in Claim 2 only in its amino acid composition at non-conserved positions.

FIG. 1

**1  2**

fusion
protein
153kDa

B-gal
114 kDa

FIG.  2

```
CAGGCGGCAAAACGGTTGGTGCCAATTACTGGTGTATTGCTATGATGCGAGTGACGTCAATAGGGTCGATTCGCGACGGTCTCCCACGTGGCCTTCCCATTGAGGTTTACGTGTTGTGTATG   -315

CGTGCCAGTGTTTTTATAACCCAAAAACTCAGCCACCACCGTCTCCACCGTACATATACTTGTCCTTCCAATTCCAGAAAACTCCTCCAATGTTGCACGATTT                      -195

TTTTACAAGAGTCATTTTGCACGTTTACAAGAATTTATTACAAGAGATTAGCTGCTTGTGTGATAAGGTCTGCACGAGATGAGAATTCAAATACGTAATGAGAGT                     -75

TTATATAGCATAATTTGCTAGGAATGTCTCTGGTTGTTGATGTTTAGGTGTTCGCTGCATTAATTATAAGACTATGTCGTACCAAAGTGATTGTACCGCTACCGCGTGGC                +46
                                                                                       M  S  Y  K  V  I  V  P  A  T  V  V  P  P  W

TCAGAGTCGGTGAGAATTGGATATTCGCAAGACACAGAGCCACCGAGGTGGGAGTCGTTCTACGGCGAACACGAAATTCGTGTACGAGCAGATTTCTCTAGGGCCGGCTTCACCCGAC       +166
 L  R  V  G  E  N  W  I  F  A  R  H  R  R  T  E  V  G  V  V  L  P  A  N  T  K  F  R  V  R  A  D  F  S  R  A  G  F  T  R

CCGTAATAGTGCGCCTCTGAACAACAACCGTAGCACTGAACGAGAAATCAACTTGAACAACGACCAATTGGAGGTGGAGCATGCGCACGAGAGTGCCTTTCGTTAGTGTTGGCTGG        +286
 P  V  I  V  R  L  L  N  N  R  S  T  E  R  E  I  N  L  N  N  D  Q  W  M  E  V  E  H  A  H  E  S  V  P  F  F  V  D  W  L

TGGGCGAAAGAACACTATGGCCGAAGTGTATTTTGAAATCGACGGACCACACATACCGCTACCGTGTGTTCAACACGAGACCCGTCGAACACTTTAAGAGCGGAGTATCGCCAAA         +406
 V  G  E  K  N  T  M  A  E  V  Y  F  E  I  D  G  P  H  I  P  L  P  V  Y  V  F  N  T  R  P  V  E  H  F  K  S  E  Y  R  Q

GTTCGTCTGGCTACTGCTTTCTATATTGGACCTGGTGGTCTGTATGTTGGTACCGCCCGCTAGCAAAAACGCTTTATTGGACGTGAACATTTCGAGCTTCATCAATTTTATAACGAAATCA   +526
 S  S  S  G  Y  C  F  L  Y  L  D  L  V  C  M  L  V  P  P  A  S  K  N  A  L  L  D  V  N  I  F  E  L  H  Q  F  Y  N  E  I

TTAATTACTATGACTGTGCGGCTTGGTCGAGGATCCATACCGCAGACACTGTCGATTCGAATTCGTGAAAGCTGATGCTGGCGTCCGGGTGCGTGTGCCT                        +646
 I  N  Y  Y  D  D  L  C  G  L  V  E  D  P  Y  A  D  T  V  D  S  N  L  P  N  K  A  A  F  V  K  A  D  A  G  G  P  G  G  A

ATTATGGACCATTTTGGACGGCCACCGGCGAGCTCAAACCTTGGTGATTACCTCAGAATATCGCCGACCAACTGGTAATTCACGAGCTGGGTCATGCATACGATTTTGTGTTTACCG     +766
 Y  Y  G  P  F  F  W  T  A  P  A  S  S  N  L  G  D  Y  L  R  I  S  P  T  N  W  M  V  I  H  E  L  G  H  A  Y  D  F  V  F  T

TCAACACTATACTCATTGAAATTTGGAACAACTCTTATGCGGATGCATCCAATACAAGTGGATGAACAAAATTAAAGACAAATTAGACGACCGACCGCAGA                       +886
 V  N  T  I  L  I  E  I  W  N  N  S  L  C  D  R  I  Q  Y  K  W  M  N  N  K  I  K  R  O  Q  L  A  R  V  Y  E  N  R  R  P  Q

AAGAGGCGACCATTCAGGCGGCTGATCGAATAACAGCCCGTTCGATAATTGGGGCTTTTTTGGAGGCTGATATATTCCGCAAAGAGGACTAGACCACATTGC                     +1006
 K  E  A  T  I  Q  A  L  I  D  N  N  S  P  F  F  D  N  W  G  F  F  E  R  L  I  I  F  T  W  L  Y  N  P  Q  R  G  L  D  T  L

GTAACATCAACCATTCGTACAGGTGCACGCGCACCCGAACTCTCTATACCGTCATGGGTAACGACTTCTCTGCCTACGACAACTTTGGTTTTATATTTTAATTTGG               +1126
 R  N  I  N  H  S  Y  R  V  H  A  T  R  N  S  S  I  P  Y  P  P  Q  I  W  S  W  L  T  T  S  A  Y  D  N  F  W  L  Y  F  N  L

TAGGCCGTGTACCCGGCAGACTTTTACGTAAACGAACAACAAAGTTGTTCATTTCAATCTACACTTGAGAGCTTTGGCGTTATCCCATTAAATATATAATTA             +1246
 V  G  V  Y  P  A  D  F  Y  V  N  E  H  H  N  K  V  V  H  F  N  L  H  L  R  A  L  A  L  G  Q  S  V  R  Y  P  I  K  Y  I  I  I

CAGACTTTGATCTGGTGAGCAAAAACTACGACATTAAACAGTATTTAGAGAGTAATTTCGATCGGATTTGTTGCGGACGACCGATTTGTTGCCGGACGTGGGTTT            +1366
 T  D  F  D  L  V  S  K  N  Y  D  I  K  Q  Y  L  E  S  N  F  D  L  V  I  P  E  E  L  R  Q  T  D  L  L  A  D  V  R  R  V  V

GTGTGATTGACGATCCGTCGCGCCACGCGAATTGTGGGCGAACCGTTTAGCGTGACGCGGACACGGAGCGAGTGTTCGAGAGTACGGTGGCCACGGACGGAAACATGTATCTGGTGGGCGTGGGGTC   +1486
 C  V  I  D  D  P  S  Q  I  V  G  E  P  F  F  S  V  Y  D  G  N  E  R  V  F  E  S  T  V  A  T  D  G  N  M  Y  L  V  G  V  G

CGGGAGTGTACGTTGCGGTGCCGCACGCGAGCAAAAACTCCATTGGCACAACTCCGCCACATTCGCCACATTGCCCAGAGAGCCCGTTCATCGCCAGAGAGCCCGT              +1606
 P  G  V  Y  T  L  R  A  P  R  G  K  N  K  R  Y  K  L  H  L  A  H  S  P  R  E  E  P  V  H  P  A  N  D  H  M  Y  L  L  V  T
```

FIG. 3

14

```
ATCCCTACTACAATCAAACGTTGACATACACACCGTACGTAAATTCTGACCTAGCCGTCGACATGGCTCATTTGTTCGGCAGCAACGATCGTAGGTATGTAGCCACGATATATTTCAATC +1726
Y  P  Y  Y  Y  N  Q  T  L  T  Y  T  P  Y  V  N  S  D  L  A  V  D  M  A  H  L  F  G  S  N  D  R  R  Y  V  A  T  I  Y  F  N
                 6

CATTCGAACAAACAGTCACCGTACATCTAAACAATATTCGTGCCGGTCGTGAAAACAACACTACCCTGTACTTTGAAATGGTAATTAGCAACCCGTTCAACGGGCAGAGCCAAACTTTCA +1846
P  F  E  Q  T  V  T  V  H  L  N  N  I  R  A  G  R  E  N  N  T  T  L  Y  F  E  M  V  I  S  N  P  F  N  G  Q  S  Q  T  F
                                                         7  8

CTATACTCGAAGACAATCCCACTTTACGACAAGGCTACTACAAATTTGACGTGGTCACGTACAGCTCCATAAGGCTGAATATGAGCGTCGCGGGTCGGCTATTATTTCGGCGATACATTT +1966
T  I  L  E  D  N  P  T  L  R  Q  G  Y  Y  K  F  D  V  V  T  Y  S  S  I  R  L  N  M  S  V  A  G  R  L  L  F  R  R  Y  I
              9                                                      10

TTGCCGGAGGTACCACCACGCTGACCATGTTCCCAAATCAAGTACTTGAGCCCAATTTGTTTCCAGACGGTTCCGCCTTGAATAGGACATTGGCACGACTAAGAGAACAGGCCGCCTTCC +2086
F  A  G  G  T  T  T  L  T  M  F  P  N  Q  V  L  E  P  N  L  F  P  D  G  S  A  L  N  R  T  L  A  R  L  R  E  Q  A  A  F
                                                                     11

TAGATAATTATTCACAACTTATGTATATTGAAAACGAGTTGCGCGACACGATTTATTTGGCCTCCCAGTTGGTAGATCCTGCGTCAGACGAATTTGTAAAGTATTATCCAGACTACTTCA +2206
L  D  N  Y  S  Q  L  M  Y  I  E  N  E  L  R  D  T  I  Y  L  A  S  Q  L  V  D  P  A  S  D  E  F  V  K  Y  Y  P  D  Y  F
      12

GAGATCCGCACACGTACGTGTACTTGTTTCGTTTCAGAGGTCTGGGTGATTTCGTGTTATTAGACTTGCAGATTGTACCATTGCTAAATTTGGCCACTGTACGTATAGCCAACATCCAAA +2326
R  D  P  H  T  Y  V  V  Y  L  F  R  F  R  G  L  G  D  F  V  L  L  D  L  Q  I  V  P  L  L  N  L  A  T  V  R  I  A  N  I  Q

ACGGTCCCCACTCGTACTTCGATACTTTGTATTTTAAAGTGGAGTTGCGCGACACAAACGGTGCGATTGTGTTTTCGTATTCGCGCCGTGGCAACGAGCCGATGACACCCGAACACCATA +2446
N  G  P  H  S  Y  F  D  T  L  Y  F  K  V  E  L  R  D  T  N  G  A  I  V  F  S  Y  S  R  R  G  N  E  P  M  T  P  E  H  H

AATTTGAAGTGTACAGTGGTTACACCGTAGAATTGTTCATGCGGGAACCCGGTAATCGATTACAATTGATTGTGAACAAAATGCTTGACACAGCGTTGCCGTCTACTCAAAACATTTTCG +2566
K  F  E  V  Y  S  G  Y  T  V  E  L  F  M  R  E  P  G  N  R  L  Q  L  I  V  N  K  M  L  D  T  A  L  P  S  T  Q  N  I  F

CTCGCATCACCGACACTCAATTAGTGGTGGGGGATACGAGCATTGAAGATAAACCTTGTAACGAGTATTAATGTAGATTGTGGCGACGACGACAACCAAAAGATAAGAGTTGTGGAAACGT +2686
A  R  I  T  D  T  Q  L  V  V  G  D  T  S  I  E  D  N  L  V  T  S  I  N  V  D  C  G  D  D  D  N  Q  K  I  R  V  V  E  T

TAAAAATGATAGCGTTCTAATAACGTTCAACAGTCAGTTATCGACTGTCGCCGCGACGACATGACACTGGTGGGTGTAGTAGTTTGCGTGCTGTTGTTATCGTCTGTAGACGGTTATTCG +2806
L  K  M  I  A  F  -  -

TTTTATTCGTCGATTGAAGCCCTGCTTTTGAACGATCGCACACAACTTTGCATAGGCGACTGTTACGAACGCAATGGCCAGCATTTGTGTGCCAGCACGTGGTCGGGATCAGAGTCTCGG +2926

TGCATAAGTGTTTTCAACAAGACCAAACACTATCGTACGGAGACTAACGGAAAATGCATAAGTAACTGTGCCAACTTCAACAACTACGCCCACGAATGGTGTGCCGTGTCCCGGTCGAAA +3046

TGGGGCCGTTGCAGCAGACGACTGGCGCTCACAGCGACACGAACACACGCCACCCACAACAAGTTCAAGACATGTG +3122
```

FIG.  3  (continued)

EP 0 502 236 A1

EP 0 502 236 A1

FIG. 4

FIG. 5a

FIG. 5 b

Virus Samples

TnGV
GVH
HaGV
EeGV
TnSNPV
AcMNPV

Mol. Wgt. Markers KDa

−205

VEF
Protein
Bands

−116
−97.4
−68

−45

−29

−20.1

−14.1

European Patent
Office

EUROPEAN SEARCH REPORT

Application Number

EP    91 10 7278
PAGE1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| P,X | JOURNAL OF GENERAL VIROLOGY vol. 72, no. 11, November 1991, GB pages 2645 - 2651; Y. HASHIMOTO ET AL.: 'Location and nucleotide sequence of the gene encoding the viral enhancing factor ot the Trichoplusia-ni granulosis virus' * Whole article * | 1-13 | C12N15/34 A01N63/00 C07K15/00 |
|  | --- |  |  |
| X | EP-A-0 384 294 (BOYCE THOMPSON INSTITUTE FOR PLANT RESEARCH, INC.) * Whole document * | 1-13 |  |
|  | --- |  |  |
| X | EP-A-0 336 341 (BOYCE THOMPSON INSTITUTE FOR PLANT RESEARCH, INC.) * Whole document * | 3-8 |  |
| D | & US-A-4973667 |  |  |
| Y |  | 1,9-10 |  |
|  | --- |  |  |
| Y | VIROLOGY vol. 141, no. 2, March 1985, NEW YORK, US pages 328 - 332; D. AKIYOSHI ET AL.: 'Cloning and sequencing of the granulin gene from Trichoplusia ni granulosis virus' * Whole article * | 1,9-10 | TECHNICAL FIELDS SEARCHED (Int. Cl.5 )  C12N C07K A01N |
|  | --- |  |  |
| D,X | VIROLOGY vol. 167, no. 1, November 1988, NEW YORK, US pages 242 - 250; A.C.G. DERKSEN ET AL.: 'Alteration of a lepidopteran peritrophic membrane by baculoviruses and enhancement of viral infectivity' * Whole article * | 3-8 |  |
|  | --- |  |  |
|  | -/-- |  |  |

The present search report has been drawn up for all claims

| Place of search | Date of completion of the search | Examiner |
|---|---|---|
| BERLIN | 19 MAY 1992 | JULIA P. |

EPO FORM 1503 03.82 (P0401)

European Patent
Office

EUROPEAN SEARCH REPORT

Application Number

EP    91 10 7278
PAGE2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | JOURNAL OF INVERTEBRATE PATHOLOGY vol. 27, no. 1, January 1976, NEW YORK, US pages 115 - 124; S. HARA ET AL.: 'Isolation and characterization of a synergistic enzyme from the capsule of a granulosis virus of the armyworm, Pseudaletia unipuncta' * Whole article * | 3 | |
| | ----- | | |
| | | | **TECHNICAL FIELDS SEARCHED (Int. Cl.5)** |

The present search report has been drawn up for all claims

| Place of search | Date of completion of the search | Examiner |
|---|---|---|
| BERLIN | 19 MAY 1992 | JULIA P. |